# EUROPÄISCHE PATENTSCHRIFT

(11) **EP 1 769 064 B1**
(45) Veröffentlichungstag und Bekanntmachung des Hinweises auf die Patenterteilung: **23.05.2012**
(21) Anmeldenummer: 05773509.4
(22) Anmeldetag: 13.07.2005
(51) Int. Cl.: C12M 1/107, C12P 5/02

(54) **BIOGASANLAGE ZUR BEREITSTELLUNG VON METHANHALTIGEN GASEN**
BIOGAS INSTALLATION FOR PRODUCING METHANE-CONTAINING GASES
INSTALLATION DE BIOGAZ POUR PREPARER DES GAZ CONTENANT DU METHANE

(30) Priorität: 16.07.2004 DE 102004035997
(43) Veröffentlichungstag der Anmeldung: 04.04.2007
(73) Patentinhaber: Patentverwaltungsgesellschaft Erweiterte Kohlenstoffnutzung bR, 48703 Stadtlohn (DE)
(72) Erfinder: Rühl, Bernhard, 89193 Blaubeuren (DE)
(74) Vertreter: Patentanwälte Ruff, Wilhelm, Beier, Dauster & Partner
(86) Internationale Anmeldenummer: PCT/EP2005/007589
(87) Internationale Veröffentlichungsnummer: WO 2006/008039

(56) Entgegenhaltungen:
- EP-A- 1 118 671
- DE-A1- 10 245 469
- DE-U1- 20 315 555
- US-A- 4 289 625

## Beschreibung

Die Erfindung betrifft Biogasanlagen zur Bereitstellung von methanhaltigen Gasen sowie ein entsprechendes Verfahren.

Die Nutzung von regenerativen Energien gewinnt zunehmend an Bedeutung, da fossile Brennstoffe in ihrer Verfügbarkeit begrenzt sind. Ein bereits etabliertes Verfahren zur Energiegewinnung ist die Biogasgewinnung. Biogas stellt einen Energieträger mit chemischer Bindungsenergie dar, dessen Hauptkomponente das Methan ist. Das Biogas entsteht durch mikrobiellen Abbau organischer Substanz (Biomasse). Dieser auch in der Natur vorkommende, in mehreren Stufen ablaufende Prozeß, findet z. B. in Sümpfen und Mooren, auf Reisfeldern, im Dickdarm von Tieren und Menschen, in Misthaufen und Jauchegruben oder auf Müllkippen statt. Aus der Biomasse, beispielsweise aus Gülle, entsteht dabei mit Hilfe von anaeroben Bakterien insbesondere Methan und Kohlendioxid. Bei der Biogasnutzung wird vor allem das entstandene Methan kontrolliert verbrannt und so z. B. zur Stromgewinnung genutzt. Hierfür wird meist das brennbare Biogas zum Antrieb von Motoren eingesetzt (beispielsweise Zündstrahlmotoren oder Gasmotoren), die über einen Generator elektrische Energie erzeugen. Es kann auch nutzbare Abwärme auf einem Temperaturniveau von ca. 80 bis 90 °C bereitgestellt werden. Von der erzeugten Strommenge werden im allgemeinen 20 bis 40 % für den Betrieb der Biogasanlage selbst verbraucht (Pumpen, Rührwerke etc.). Von der erzeugten Wärmemenge werden etwa 30 bis 50 % für die Heizung des oder der Reaktoren eingesetzt. Zum Teil wird Biogas auch nur zur reinen Wärmeerzeugung genutzt.

An dem Prozeß der Biogasentstehung, also der Vergärung von organischen Substraten, sind vielfältige Organismenstämme beteiligt. Deren Zusammensetzung ergibt sich vor allem aus den spezifischen Prozeßbedingungen, wobei insbesondere die Ausgangsstoffe der Vergärung, die Temperatur, der pH-Wert etc. eine Rolle spielen. Da sich die Mikroorganismenzusammensetzung an verschiedene Substrate anpassen kann, ist fast jede organische Substanz durch Vergärung abbaubar. Die herkömmlicherweise für die Biogasproduktion genutzten organischen Stoffe sind meistens Rest- oder Nebenprodukte aus verschiedenen Bereichen. So kommen beispielsweise in der Landwirtschaft Reststoffe der Pflanzenproduktion, Festmist oder Flüssigmist (Gülle) für die Biogasproduktion zum Einsatz. Die Hauptkomponenten der Ausgangsstoffe für die Biogaserzeugung werden im wesentlichen von Kohlenhydraten, Eiweißen, Fetten, Zellulosen und Hemizellulosen gebildet. Nicht geeignet sind jedoch Materialien, die Lignin und lignininkrustierte Zellulose, d. h. also die strukturgebenden Komponenten von Holz und Stroh, enthalten. Die Verwendung von Holz und Stroh für die Biogasproduktion ist daher bisher nicht möglich.

Herkömmliche Anlagen zur Biogaserzeugung in der Landwirtschaft verwenden oftmals die bei der Tierhaltung anfallende Gülle oder Mist. Die Gülle bzw. der Mist gelangt zum Vergären in einen Gärbehälter (Reaktor), wobei gegebenenfalls weitere Cosubstrate zugesetzt werden. Hier verweilt das Substrat mehrere Tage, wobei durch die Aktivität der Mikroorganismen Biogas gebildet wird. Durch ständiges Rühren wird die Bildung von Schwimmdecken und Sinkschichten verhindert und das Entweichen der entstehenden Gase erleichtert. Das Biogas wird abgezogen und seiner weiteren Verwertung, also insbesondere einer Verbrennung, zugeführt. Das vergorene Substrat gelangt in einen Schlammbehälter als Endlager und kann als Düngemittel verwendet werden.

Das eigentlich nutzbare Potential des Biogases liegt in dessen Methangehalt. Die in der Praxis erzielten Methanerträge hängen wesentlich von den eingesetzten Substraten sowie der tatsächlichen Abbauleistung der jeweiligen Biogasanlage ab. Es ist jedoch zu beobachten, daß die erzielten Erträge weit unterhalb der theoretisch möglichen Werte liegen.

Aus der EP 1 118 671 A1 geht eine Biogasanlage mit einem aeroben Fermentationsreaktor, einer Verschwelungseinrichtung sowie einem anaeroben Fermentationsreaktor hervor. Die DE 203 15 555 U1 betrifft eine Vorrichtung zum Verwerten von Flüssigabfällen, wobei die Vorrichtung eine Belebungsanlage, Fermentieranlage und eine Pyrolyseeinheit umfasst. Die DE 102 45 469 A1 offenbart ein Verfahren zum Erzeugen elektrischer und thermischer Energie aus biogenen Reststoffen unter Verwendung einer Biogasanlage und eines Pyrolysereaktors. In der US 4,289,625 wird ein Verfahren zur anaeroben Vergärung sowie Vergasung von biologischem Material beschrieben.

Die Erfindung stellt sich daher die Aufgabe, die Qualität des bei der Vergärung von organischen Substraten erzeugten Biogases zu verbessern und insbesondere den Methangehalt zu steigern, wodurch das erzielte Biogas ein verbessertes Energiepotential erlangen soll. Weiterhin stellt sich die Erfindung die Aufgabe, eine Biogasanlage bereitzustellen, bei welcher in herkömmlichen Biogasanlagen nicht verwertbare Substrate eingesetzt werden können.

Diese Aufgabe wird durch eine Biogasanlage gelöst, wie sie im Anspruch 1 beschrieben ist. Bevorzugte Ausführungsformen dieser Biogasanlage sind in den Ansprüchen 2 bis 16 ausgeführt. Anspruch 17 befaßt sich mit einem Verfahren zu Herstellung von methanhaltigen Gasen aus organischen Substraten. Die Ansprüche 18 bis 22 betreffen bevorzugte Ausführungsformen dieses Verfahrens. Der Wortlaut sämtlicher Ansprüche wird hiermit durch Bezugnahme zum Inhalt der Beschreibung gemacht.

Die erfindungsgemäße Biogasanlage zur Bereitstellung von methanhaltigen Gasen aus insbesondere organischen Substraten umfaßt mindestens einen Reaktor. Dieser im folgenden als Reaktor I bezeichnete Reaktor weist mindestens eine Gaszufuhreinheit für die Zufuhr von kohlenmonoxid- und wasserstoffhaltigem Gas auf. In dem Reaktor findet die Vergärung der organischen Substrate durch anaerobe Mikroorganismen, insbesondere durch Bakterien, statt. Im Zuge dieser Vergärung bzw. der Verstoffwechselung der Substrate wird von den Mikroorganismen methanhaltiges Gas, insbesondere Biogas, gebildet, welches von den Mikroorganismen freigesetzt und/oder gespeichert wird. Das gespeicherte Biogas bzw. Methan wird bei einem Absterben bzw. einer Zerstörung der Mikroorganismen freigesetzt. Neben dieser Biogasproduktion durch die Mikroorganismen ist es erfindungsgemäß vorgesehen, daß kohlenmonoxid- und wasserstoffhaltige Gas zusätzlich eingespeist wird. Das kohlenmonoxid- und wasserstoffhaltige Gas kann beispielsweise über entsprechende Vorratsbehälter, beispielsweise Gasflaschen, dem Reaktor zugeführt werden. Kohlenmonoxid und Wasserstoff werden von den Mikroorganismen ebenfalls zu Methan verstoffwechselt und letztendlich freigesetzt. Durch diese verschiedenen Stoffwechselvorgänge wird daher ein Biogas produziert, welches einen besonders hohen Gehalt an Methan im Vergleich mit herkömmlich hergestellten Biogasen aufweist und daher für die Energiegewinnung aus dem erfindungsgemäß hergestellten Biogas sehr vorteilhaft ist.

Als organische Substrate im Reaktor eignen sich alle herkömmlicherweise in Biogasanlagen eingesetzten Substrate. Mit besonderem Vorteil können beispielsweise flüssiger Mist oder Gülle eingesetzt werden. Doch auch andere Substrate, wie beispielsweise Grüngut oder organische Küchenabfälle, sind gleichermaßen geeignet. Es können auch verschiedene Substrate miteinander kombiniert werden.

Bei der Gaszufuhreinheit handelt es sich um eine Pyrolysegaszufuhreinheit. Besonders bevorzugt ist eine Holzgaszufuhreinheit. Geeignete Pyrolysegase, die mit der entsprechenden Zufuhreinheit dem Reaktor I zugeführt werden, enthalten kohlenmonoxid- und wasserstoffhaltiges Gas. Dieses Gasgemisch kann neben Kohlenmonoxid und Wasserstoff auch andere Gase enthalten. Insbesondere kann das Gas vorzugsweise durch eine Pyrolyse verschiedener Substrate gebildet werden. Ein besonders bevorzugtes Beispiel einer solchen Pyrolyse ist die Vergasung von Holz und/oder Borke, bei welcher das sogenannte Holzgas entsteht.

Der Holzvergasungsprozeß beruht auf dem Effekt der unvollständigen Verbrennung. Im Gegensatz zur normalen, vollständigen Verbrennung, bei welcher ausreichend Sauerstoff vorhanden sein muß, wird bei der Holzgaserzeugung Sauerstoff im unterstöchiometrischen Verhältnis zugegeben. Statt der Oxidationsprodukte Kohlendioxid und Wasserdampf, die bei einer vollständigen Verbrennung von Holz entstehen würden, entsteht bei der Holzvergasung im wesentlichen ein Gemisch aus Kohlenmonoxid und Wasserstoff sowie auch Kohlendioxid und Wasserdampf. Man spricht in diesem Zusammenhang auch von einer Verschwelung. Das entstehende kohlenmonoxid- und wasserstoffhaltige Gas, das Holzgas, ist in besonderer Weise zur Einspeisung in den Reaktor I der erfindungsgemäßen Biogasanlage geeignet. Hierfür eignen sich jedoch auch andere Pyrolysegase, die bei der Verschwelung anderer Substrate entstehen, wie beispielsweise bei der Verschwelung von Stroh, Knochen, Kunststoffen, beispielsweise PVC, Autoreifen, Plastikbechern oder Ähnlichem. Grundsätzlich sind alle pyrolysierbaren Substrate mit geringem Stickstoffgehalt zur Bereitstellung des kohlenmonoxid- und wasserstoffhaltigen Gases durch Pyrolyse geeignet, die vorzugsweise verhältnismäßig lange Kohlenwasserstoffketten aufweisen.

In einer besonders bevorzugten Ausführungsform der erfindungsgemä-βen Biogasanlage handelt es sich bei der Gaszufuhreinheit um einen Pyrolysator, innerhalb dessen die eben beschriebene Pyrolyse stattfindet. Dieser Pyrolysator weist mindestens eine Pyrolysegasleitung auf, die zum Reaktor I führt und diesem das kohlenmonoxid- und wasserstoffhaltige Gas zuleitet. In besonders bevorzugter Weise ist der Pyrolysator ein Holzvergaser mit mindestens einer Holzgasleitung, die zum Reaktor I führt.

In Weiterbildung weist der Pyrolysator mindestens eine Zufuhreinrichtung für sauerstoffhaltiges Gas auf. Hierbei kann es sich beispielsweise um reinen Sauerstoff handeln, der aus einer entsprechenden Gasflasche oder einer anderen Gasquelle zugeführt wird. Vorzugsweise handelt es sich bei dem sauerstoffhaltigen Gas um Luft, die im Sauerstoffgehalt angereichert ist (aufbereitete Luft). Für eine solche Anreicherung der Luft mit Sauerstoff können übliche Vorrichtungen verwendet werden, wie sie beispielsweise aus der Nahrungsmittelindustrie zur Bereitstellung von Schutzgasen bekannt sind. Durch die Zufuhr von sauerstoffhaltigem Gas bzw. von im Sauerstoffgehalt angereicherter Luft bei der Verschwelung der Substrate im Pyrolysator wird erreicht, daß der Stickstoffgehalt im resultierenden kohlenmonoxid- und wasserstoffhaltigen Gas, beispielsweise im Holzgas, vermindert bzw. deutlich reduziert ist. Der Stickstoffgehalt herkömmlicher Pyrolysegase bzw. Holzgase würde sich nachteilig auf das Wachstum und/oder den Stoffwechsel der Mikroorganismen im Reaktor I auswirken. Daher ist es besonders vorteilhaft, diesen Stickstoffgehalt des Pyrolysegases durch die Zufuhr von sauerstoffhaltigem Gas bzw. durch die Zufuhr von im Stickstoffgehalt reduziertem Gas zu vermindern. Normale Luft enthält neben einigen weiteren Bestandteilen im wesentlichen neben 20 % Sauerstoff etwa 80 % Stickstoff (jeweils Vol.-%). Besonders bevorzugt ist es, wenn mit der Zufuhreinrichtung ein sauerstoffhaltiges Gas mit einem Sauerstoffgehalt von 70 % oder mehr dem Pyrolysator zugeführt wird. Allerdings ist zu beachten, daß der Sauerstoffgehalt in der Brennkammer bzw. dem Brennbereich des Pyrolysators nicht zu hoch sein sollte. An dieser Stelle ist ein Sauerstoffgehalt von etwa 50 % oder weniger bevorzugt. Besonders bevorzugt ist ein Sauerstoffgehalt in der Brennkammer von etwa 20 %. Das Problem hierbei ist, daß bei einem zu hohen Sauerstoffgehalt die Reaktionstemperatur sehr hoch wird und unter Umständen eine vollständige Verbrennung stattfindet, die den Pyrolysator schädigen könnte. Daher sind Brenntemperaturen unterhalb von etwa 1.000 °C bevorzugt. Besonders bevorzugt sind Temperaturen im Brennbereich von etwa 600 °C. In bevorzugter Weise wird daher die Temperatur im Pyrolysator, insbesondere im Brennbereich des Pyrolysators, kontrolliert und vorzugsweise gesteuert und/oder geregelt.

Der Pyrolysator weist mindestens eine weitere Gaszuleitung, insbesondere eine Biogaszuleitung, auf. Durch diese weitere Gaszufuhr wird zum einen eine interne Kühlung innerhalb des Pyrolysators ermöglicht. Zum anderen wird hierdurch die Zusammensetzung des Gases, welches der Brennkammer des Pyrolysators zugeführt wird, beeinflußt. Insbesondere durch die Zuleitung von Biogas zusammen mit der im Sauerstoffgehalt angereicherten Luft kann also ein Gas bereitgestellt werden, was im Stickstoffgehalt deutlich reduziert ist und darüber hinaus einen für die Vergasung bzw. Verschwelung der Substrate im Pyrolysator einen besonders geeigneten Sauerstoffgehalt aufweist. Beispielsweise kann etwa ein Drittel des der Brennkammer zugeführten Gasvolumens aus aufbereiteter Luft mit einem Sauerstoffgehalt von etwa 70 % und zwei Drittel des Volumens aus Biogas bestehen. Dies entspricht im Ergebnis etwa der ursprünglichen Sauerstoffkonzentration der Luft (20 %). Neben Biogas sind auch andere Gase in diesem Zusammenhang geeignet. Neben der Zufuhr des sauerstoffhaltigen Gases und der Zufuhr des weiteren Gases spielt beispielsweise auch die Größe und Zusammensetzung des zu verschwelenden Substrates im Pyrolysator, beispielsweise Holzhackschnitzel oder Ähnliches, für den Lufteintrag bzw. den Sauerstoffgehalt eine Rolle.

Ein wichtiger Vorteil der erfindungsgemäßen Biogasanlage ist, daß durch die Reduzierung des Stickstoffanteils im Gas, welches für die Pyrolyse, beispielsweise die Holzvergasung, zugeführt wird, der Stickstoffgehalt des Pyrolysegases, welches im Reaktor I durch Mikroorganismen weitere verstoffwechselt wird, reduziert wird. Hierzu tägt auch die Verwendung von Pyrolysesubstraten bei, die verhältnismäßig arm an Stickstoff sind. Ein erhöhter Stickstoffanteil in dem Pyrolysegas wäre schädlich für die Population der Mikroorganismen im Reaktor. Durch die Reduzierung des Stickstoffanteils im gesamten System wird daher die Produktion eines besonders hochwertigen Biogases bzw. eines besonders energiereichen Biogases mit hohem Methangehalt ermöglicht. Darüber hinaus wird hierdurch die Verwendung von Substraten, insbesondere von Holz o. ä., für die Biogasproduktion ermöglicht, was mit herkömmlichen Biogasanlagen nicht möglich ist.

In einer besonders bevorzugten Ausführungsform sind dem Pyrolysator Steuerungs- und/oder Regelungsmittel zur Steuerung und/oder Regelung der Sauerstoffkonzentration und/oder der Temperatur im Pyrolysator zugeordnet. Durch diese Steuerungs- oder Regelungsmittel wird vorteilhafterweise gewährleistet, daß die Temperatur bei der Verschwelung der Substrate nicht zu hoch wird und es zu einer Schädigung der Anlage kommen könnte. In besonders bevorzugter Weise werden hierfür ein oder mehrere Temperaturfühler als Sensoren eingesetzt, die beispielsweise die Sauerstoffzufuhr und/oder die weitere Gaszufuhr mittels geeigneter Steuerprozessoren, Steuerleitungen und/oder Steuerelemente steuern oder regeln.

In einer besonders bevorzugten Ausführungsform der erfindungsgemä-βen Biogasanlage ist an der Gaszuleitung des Pyrolysators, insbesondere an der Biogaszuleitung, mindestens ein Verdichter angeordnet. Mit Hilfe eines solchen Verdichters kann das Gas, insbesondere das Biogas, mit Überdruck in den Pyrolysator eingespeist werden. Dies ist insbesondere im Hinblick auf die Kühlwirkung des eingespeisten Gases vorteilhaft. Als Verdichter kommen alle Arten herkömmlicher Verdichter zur Druckerhöhung des Gases in Frage. Beispielsweise können Kompressoren oder Vergleichbares eingesetzt werden. Besonders vorteilhaft ist die Verwendung von Seitenradverdichtern. Auch an anderen Stellen der erfindungsgemäßen Biogasanlage können Verdichter mit Vorteil vorgesehen sein. Beispielsweise kann das im Reaktor I entstehende Gas über eine entsprechende Gasleitung in ein Endlager überführt werden, wobei die Gaseinleitung mit Hilfe eines Gasrührwerkes erfolgen kann, welchem ein entsprechender Verdichter vorgeschaltet sein kann.

In einer weiteren bevorzugten Ausführungsform der erfindungsgemäßen Biogasanlage befindet sich im Bereich der Pyrolysegasleitung des Pyrolysators eine Wassereinspritzungseinrichtung und/oder ein Kondensatfänger. Diese Wassereinspritzung in der Gasleitung dient vor allem dem Abfangen von nicht ausgekoksten Substratschwebstoffen, insbesondere Holzschwebstoffen, aus der Pyrolyse, die im Gasstrom mitgerissen werden. Besonders bevorzugt ist es, daß die Wassereinspritzung mit hohem Druck erfolgt, beispielsweise mit Hochdruck (z. B. 300 bar bei 60 l/h), so daß ein Wassernebel entsteht, in welchem die Schwebstoffpartikel zerreißen oder sich so mit Wasser vollsaugen, daß sie über einen Kondensatfänger aus dem Gasstrom entfernt werden können. Die Flüssigkeit im Kondensatfänger kann mit besonderem Vorteil in die Pyrolyseeinheit, insbesondere in die Brennkammer, zurückgepumpt werden. Hierdurch wird zum einen erreicht, daß das nicht ausgekokste Material erneut für die Pyrolyse zur Verfügung steht. Zum anderen kann hierdurch eine Kühlwirkung erzielt werden, die darüber hinaus eine weitere Kontrollmöglichkeit der Temperatur im Brennbereich ermöglicht. Vorteilhafterweise kann auch eine zusätzliche Wasserkühlung vorgesehen sein, die beispielsweise mit der Wassereinspritzung, dem Kondensatfänger und/ oder einer geeigneten Pumpe kombiniert bzw. gekoppelt sein kann. Eine entsprechende Kühleinrichtung kann jedoch auch unabhängig von der Wassereinspritzung und/oder dem Kondensatfänger ausgebildet sein. Beispielsweise kann eine Flüssigkeitskühleinrichtung mit einer möglicherweise unabhängigen Pumpe der Brennkammer zugeordnet sein. Die Temperatur wird auch durch den Massenstrom der Substrate in der Brennkammer beeinflußt. Es kann daher auch bevorzugt sein, daß die Pyrolysesubstrate selbst gekühlt werden.

Eine Regulierung bzw. Steuerung des Pyrolysators bzw. des Holzvergasers kann über die Biogaseinblasung, die Einblasung an sauerstoffhaltigem Gas, dem Input der Biomasse, insbesondere des Holzes, über Wasserstrahl und/oder Unterdruck erfolgen.

Dem Pyrolysator ist ein weiterer Reaktor II vorgeschaltet, welcher mindestens eine Gasleitung zum Pyrolysator aufweist. Das in dem Reaktor II entstehende Gas, insbesondere Biogas, wird durch diese Gasleitung als weiteres Gas dem Pyrolysator zugeführt. Durch dieses zugeführte Gas wird in der eben beschriebenen Weise die Gaszusammensetzung und die Temperatur im Pyrolysator beeinflußt. Die erfindungsgemäße Biogasanlage kann also so ausgestaltet sein, daß ein Reaktor II, in welchem durch die Vergärung von organischen Substraten Biogas entsteht, über eine Gasleitung mit einem Pyrolysator, insbesondere einem Holzvergaser, kombiniert ist. In diesem Pyrolysator wird einerseits Biogas, hauptsächlich bestehend aus Methan und Kohlendioxid, sowie andererseits im Sauerstoffgehalt angereicherte Luft der Brennkammer zugeführt. In der Brennkammer findet die Verschwelung von Substraten, insbesondere von Holz, statt. Das Kohlendioxid reagiert hierbei mit dem Kohlenstoff beispielsweise im Holz zu Kohlenmonoxid, im selben Verhältnis wird Wasserstoff frei. Das Methan aus dem Biogas wird im wesentlichen unbeinflußt durch den Pyrolysator hindurchgeführt. Das resultierende Kohlenmonoxid und der Wasserstoff sowie das Methan aus dem Biogas werden einem weiteren Reaktor I zugeführt, wobei in diesem Reaktor Kohlenmonoxid und Wasserstoff zu Methan und Kohlendioxid in Verbindung mit Wasser und Wasserstoff verstoffwechselt werden. Hierdurch wird ein Gas produziert, was einen ausgesprochen hohen Methangehalt hat und für die weitere Energiegewinnung sehr vollteilhaft ist.

Bevorzugterweise herrscht im Reaktor II eine Temperatur von etwa 40 °C, im Reaktor I eine höhere Temperatur, etwa 50 bis 60 °C, insbesondere etwa 55 °C. Durch diese unterschiedlichen Reaktortemperaturen stellt sich in beiden Reaktoren jeweils eine unterschiedliche Mikroorganismenpopulation ein, was für eine optimale Verwertung der Substrate sehr vorteilhaft ist. Durch die Einleitung der Gase aus dem Pyrolysator in den Reaktor I stellt sich diese erhöhte Temperatur im Reaktor I ohne externe Heizeinwirkung ein, was einen besonderen Vorteil der Erfindung darstellt.

Mit besonderem Vorteil werden die Gase aus dem Pyrolysator mit Unterdruck in den Reaktor I eingeführt. Dies kann vorzugsweise so realisiert sein, daß dem Reaktor I mindestens eine Druckleitung für die Substratzufuhr zugeordnet ist, wobei vorzugsweise die Druckleitung mindestens eine Gaseinspeisung für kohlenmonoxid- und wasserstoffhaltiges Gas, also insbesondere für das Gas aus dem Pyrolysator, aufweist. Durch die Unterdruckzuleitung der Gase und insbesondere auch durch einen Unterdruck im Reaktor I selbst wird eine gute Verteilung bzw. Lösung von insbesondere Kohlenmonoxid im Reaktor I erzielt. Die Druckverhältnisse im Bereich der Substratdruckleitung können beispielsweise vor der Druckleitung bei etwa 12 bar und danach bei etwa 0,5 bar im Unterdruckbereich liegen. Eine gute Verteilung von Kohlenmonoxid im Reaktor I ist für die Methanbildung durch die Mikroorganismen sehr vorteilhaft. Diese gute Verteilung kann weiterhin mit Vorteil durch ein Rührwerk im Reaktor I gefördert werden. Mit Vorteil befindet sich ein solches Rührwerk in etwa der mittleren Höhe des Reaktors. Vorzugsweise handelt es sich um ein kontinuierlich rührendes Rührwerk. Durch diese verschiedenen Maßnahmen wird das Durchperlen der Gase und eine Lösung von Kohlenmonoxid im Reaktor I gefördert. Darüber hinaus ist eine gute Verteilung der heißen Gase aus dem Pyrolysator vorteilhaft, um keine Schäden an den Materialien der Biogasanlage durch lokale Überhitzung zu verursachen.

In einer besonders bevorzugten Ausführungsform der erfindungsgemä-βen Biogasanlage ist der Biogasanlage mindestens ein Gasspeicherungs- und/oder -entmischungsbehältnis zugeordnet. Vorzugsweise kann ein solches Gasbehältnis mit Zuleitungen aus einem oder mehreren Reaktoren und mit Ableitungen zum Pyrolysator, insbesondere über einen Verdichter, versehen sein. Weiterhin können Ableitungen aus dem Gasbehältnis zu Gasrührwerken vorgesehen sein, die beispielsweise für die Rührung eines Reaktors II und/oder die Rührung eines Endlagers vorgesehen sind. Weiterhin können für entsprechende Gasrührwerke auch separate Gasbehältnisse bzw. Gasspeicherungs- und/oder -entmischungsbehältnisse vorgesehen sein. Auch ausgehend von einem Endlager kann eine Zuleitung zu einem zentralen Gasbehältnis und/oder einem weiteren Gasbehältnis vorgesehen sein.

Mit besonderem Vorteil handelt es sich bei diesem Gasspeicherungs- und/oder -entmischungsbehältnis um einen Gassack. Ein Gassack kann lose aufgehängt sein und beispielsweise als im wesentlichen geschlossener flexibler Schlauch gestaltet sein, der beispielsweise aus einer Kunststoffplane gefertigt ist. Andererseits kann als Gasspeicherungs- und/oder -entmischungsbehältnis auch ein mehr oder weniger festes Gefäß bzw. eine feste Ummantelung mit einer inneren flexiblen Hülle vorgesehen sein. Die Gestaltung als flexibler Gassack hat jedoch den Vorteil, daß hierdurch mit sehr geringem Materialaufwand eine kostengünstige Gasspeicherung sowie auch eine kostengünstige Gasentmischung bzw. Gastrennung ermöglicht wird. Die Aufhängung eines solchen Gassacks kann beispielsweise in einem Futtersilo erfolgen.

Mit besonderem Vorteil weist das Gasbehältnis, insbesondere der Gassack, mindestens eine Zuleitung für die Zufuhr von Gasen auf, die vorzugsweise in etwa der mittleren Höhe des Gasbehältnisses endet. Durch die unterschiedlichen Eigenschaften der Komponenten des eingespeisten Gases im Behältnis beruhigt und entmischt sich das zugeführte Gas innerhalb des Behältnisses. So erhöht sich im oberen Teil des Behältnisses der Methangehalt. Im unteren Teil des Behältnisses erhöht sich der Kohlendioxidanteil. Vorzugsweise weist das Behältnis eine Ableitung zu einem weiteren Gasspeicher und/oder zu einem Gasverbraucher, beispielsweise einem Motor, auf. Aufgrund des hohen Methangehalts des Gases im oberen Bereich des Behältnisses ist in diesem Bereich das Gas energetisch besonders vorteilhaft, so daß erfindungsgemäß mindestens eine Ableitung zu einem Gasspeicher und/oder einem Gasverbraucher im oberen Bereich des Behältnisses vorgesehen ist. Durch die Anreicherung von Kohlendioxid im unteren Bereich des Behältnisses ist diese Gaszusammensetzung aufgrund ihrer Pufferkapazität in wäßriger Lösung mit besonderem Vorteil für eine Pufferung von Reaktoren geeignet, so daß das Behältnis vorzugsweise eine Ableitung zu mindestens einem Reaktor aufweist, welche sich insbesondere im unteren Bereich des Behältnisses befindet. Hierbei handelt es sich mit Vorteil beispielsweise um eine Ableitung zum Reaktor II. Der pH-Wert innerhalb der Reaktoren sollte sich aufgrund der Biologie der Mikroorganismenpopulationen vorzugsweise im Bereich zwischen etwa pH 5,8 bis 8,3 bewegen. Für diesen pH-Bereich ist eine solche Pufferung mit Kohlendioxid, insbesondere in wäßriger Lösung, sehr geeignet. Eine Pufferung der Reaktoren kann alternativ hierzu oder in Kombination auch mit anderen, üblichen Methoden erfolgen, beispielsweise durch die Zugabe von Kalk und/oder Propionsäure. Weiterhin kann das Gasgemisch aus dem unteren Behältnisbereich mit hohem Kohlendioxidanteil mit Vorteil zum Betreiben von Gasrührwerken eingesetzt werden, die beispielsweise zur Rührung von Reaktoren und/oder Endlagern verwendet werden können. In einer besonders bevorzugten Ausführungsform des Gasbehältnisses sind innerhalb des Behältnisses befeuchtete flexible Materialien, insbesondere Tücher, angeordnet. Diese Tücher sind vorzugsweise lose aufgehängt. Hierdurch wird eine Entmischung des Gases innerhalb des Behältnisses gefördert. Diese Materialien können beispielsweise aus Goretexmembranen und/oder Leinen oder Ähnlichem gebildet sein. Mit besonderem Vorteil findet eine Befeuchtung der Materialien durch Wassereinspritzung in den Gassack unter Hochdruck statt. Durch eine derart gezielte Vernebelung wird vorteilhafterweise das im Gasgemisch enthaltene Kohlendioxid angelöst, welches sodann als kohlensäurehaltiges Wasser in den unteren Bereich des Gasbehältnisses ablaufen kann. Dieses kohlensäurehaltige Wasser mit Pufferkapazität kann zur Pufferung von Reaktoren mit Vorteil eingesetzt werden.

In einer weiteren bevorzugten Ausführungsform der erfindungsgemäßen Biogasanlage ist zur Absperrung von Gasströmen mindestens ein Siphon vorgesehen, vorzugsweise ist ein solcher Siphon mit Wasser flutbar. Mit Hilfe solcher Siphons können Gasstrecken ohne weitere Schieber abgesperrt werden. Dies ist besonders vorteilhaft, da herkömmliche Schieber von den Gasen, insbesondere von Biogas, angegriffen werden können und sehr schnell korrodieren würden. Die erfindungsgemäß verwendeten Siphons sind vor allem für die Absperrung von Gasleitungen im niedrigen Druckbereich geeignet, welche beispielsweise einen Druck entsprechend einer 3 cm Wassersäule aufweisen. Erfindungsgemäß schließt sich am tiefsten Punkt der im Siphon geführten Gasleitung eine Wassersäule in entsprechender Leitung an. Zum Absperren der Gasleitung wird die Wassersäule in der siphonförmigen Gasleitung beispielsweise durch Schaltung entsprechender Ventile und/oder durch Abschalten einer nachgeschalteten Wasserpumpe oder durch aktives Pumpen von Wasser erhöht und so der Gasfluß verschlossen. Hierfür reicht beispielsweise eine Wassersäule von 3,5 cm bei entsprechend niedrigem Druck im Gasbereich. Diese Siphons erfüllen also eine Sicherheitsfunktion, da hiermit bei Gefahr der Gasfluß unterbrochen werden kann. Gleichzeitig kann der beschriebene Siphon zusätzlich oder alternativ dazu zur Gastrocknung genutzt werden, indem die Wassersäule unterhalb der siphonförmigen Gasleitung als Kondensatabscheider wirkt und Wasser aus dem Gasstrom herauszieht. Unabhängig von den beschriebenen Siphons weist die erfindungsgemäße Biogasanlage vorteilhafterweise weitere Sicherheitsventile auf, welche die Gasleitungen bei Gefahr absperren können.

In einer besonders bevorzugten Ausführungsform weist die erfindungsgemäße Biogasanlage mindestens einen heizbaren und/oder kühlbaren Substratvorratsbehälter auf. Insbesondere der Einsatz von einem zentralen Substratvorratsbehälter ist bevorzugt. Hierdurch ist nur eine zentrale Heizung erforderlich, was aus Kostengesichtspunkten für den Bau, den Betrieb und die Wartung der Biogasanlage sehr vorteilhaft ist. Aus diesem vorzugsweise zentralen Substratvorratsbehälter bzw. diesem Anmisch- und Heizbehälter wird das Substrat, beispielsweise Gülle, auf die verschiedenen Reaktoren verteilt. Dies kann insbesondere mit Hilfe einer zentralen Pumpe und/oder einem Substrat- bzw. Gülleverteiler vorgenommen werden.

In dem mindestens einen Substratvorratsbehälter, insbesondere dem zentralen Substratvorratsbehälter, kann das Substrat erhitzt werden, bevor es auf den oder die Reaktoren verteilt wird. Besonders bevorzugt ist eine Erhitzung auf bis zu etwa 130 °C, da hierdurch eventuell im Substrat enthaltene Krankheitserreger, wie beispielsweise Salmonellen oder Ähnliches, abgetötet werden. Besonders bevorzugt ist es, daß das Substrat über einen längeren Zeitraum, beispielsweise über etwa 5 bis 10 Minuten, vorzugsweise etwa 7 Minuten, oder auch länger, erhitzt wird, wodurch zuverlässig enthaltene Krankheitserreger bzw. Keime abgetötet werden. Man spricht hier auch von einer Hygienisierung des Substrates. Ein weiterer Vorteil der Erhitzung des Substrates ist, daß es durch die Überhitzung zu einer Spaltung von Inhaltsstoffen des Substrates kommt, wodurch die Umsetzung der Substrate durch die Mikroorganismen in den Reaktoren erleichtert und beschleunigt wird. Dies ermöglicht beispielsweise verkürzte Verweilzeiten der Substrate in den Reaktoren.

In besonders bevorzugter Weise steht der mindestens eine Substratvorratsbehälter mit einem Reaktor, insbesondere dem Reaktor II, über eine Gasausgleichsleitung in Verbindung. Durch diese Gasausgleichsleitung, insbesondere eine Gasausgleichsdruckleitung, kann der Flüssigkeitsstand des Reaktors den Druck und/oder die Temperatur in dem Substratvorratsbehälter zumindest mitbestimmen. Hierdurch wird eine Art Ventilfunktion ausgeübt, so daß die Temperatur auf 130 °C oder weniger gehalten werden kann. Diese Obergrenze von 130 °C ist besonders vorteilhaft, da bei einem Überschreiten dieser Temperatur für die Biogasanlage bestimmte Rechtsvorschriften, insbesondere die Dampfkesselverordnung, mit entsprechenden Auflagen einschlägig wären.

Eine Erhitzung des Substrates im Substratvorratsbehälter erfolgt vorzugsweise während oder nach einer Beschickung des Behälters mit frischem Substrat. Dies kann ein- oder mehrmals pro Tag erfolgen und hängt selbstverständlich von der Dimensionierung der Anlage ab. Bei entsprechender Auslegung kann die Beschickung auch seltener vorgenommen werden bzw. häufiger erforderlich sein. Der Substratlauf erfolgt vorzugsweise mit Hilfe einer zentralen Pumpe, wobei es sich hierbei beispielsweise um eine kontinuierlich arbeitende Pumpe handeln kann.

Bevorzugterweise ist der mindestens eine Substratvorratsbehälter, insbesondere der heizbare Substratvorratsbehälter, kühlbar. Die Prozessierung von heißem Substrat, insbesondere von Substraten oberhalb von 80 °C, stellt besondere Anforderungen an die verbauten Materialien der erfindungsgemäßen Biogasanlage. Daher ist es bevorzugt, die Substrate im Substratvorratsbehälter vor der weiteren Einleitung in die Anlage abzukühlen, um eine Schädigung der Anlage zu vermeiden bzw. um keine besonderen Anforderungen an das zu verbauende Material der Anlage stellen zu müssen. Vorzugsweise erfolgt die Kühlung des erhitzten Substrates durch einen Rückfluß von bereits im System befindlichen Substraten, beispielsweise mit Substraten aus dem oder den Reaktoren bzw. aus einem Endlager. Dies erfolgt vorzugsweise über einen oder mehrere Substrat-, insbesondere Gülleverteiler. Eine Rückkühlung des Substrats erfolgt bevorzugt auf etwa 70 bis 80 °C.

Um eine ausreichende Durchmischung in dem oder den Reaktoren zu gewährleisten, sind vorzugsweise ein oder mehrere Rührwerke in den Behältnissen der erfindungsgemäßen Biogasanlage vorgesehen. Im Reaktor I ist insbesondere mindestens ein Rührwerk angeordnet, welches sich vorzugsweise in der mittleren Höhe des Reaktors befindet. Dieses Rührwerk rührt vorzugsweise kontinuierlich, wodurch insbesondere ältere Mikroorganismen durch die entstehenden Scherkräfte zerstört werden und im weiteren als Biomasse zum Abbau durch andere Mikroorganismen zur Verfügung stehen. Das innerhalb der Mikroorganismen gespeicherte Gas wird hierbei vorteilhafterweise freigesetzt. Darüber hinaus kann mit einem solchen kontinuierlichen Rührwerk ein Unterdruck im Reaktor erzeugt oder gefördert werden, welcher für die Verteilung und Lösung der in den Reaktor eingespeisten Gase vorteilhaft sein kann. Der Reaktor II weist vorzugsweise ein Rührwerk im unteren Bereich und/oder ein Rührwerk im oberen Bereich des Reaktors auf. Vorzugsweise handelt es sich hierbei um Rührwerke, die in Abständen rühren, beispielsweise alle 20 Minuten. Durch das Rührwerk im oberen Bereich werden vorteilhafterweise durch die entstehende Scherkräfte Mikroorganismen bzw. Bakterien zerstört und das von den Bakterien produzierte und in deren Außenhülle gespeicherte Gas freigesetzt. Die abgetöteten Mikroorganismen selbst stehen wiederum als Biomasse zur weiteren Verstoffwechselung zur Verfügung. Das Rührwerk im unteren Bereich des Reaktors sorgt vornehmlich für die Vermeidung von Sinkschichten und für eine gleichmäßige Durchmischung des Reaktorinhalts.

In besonders bevorzugter Weise ist das Rührwerk im unteren Bereich des Reaktors II ein Gasrührwerk. Mit Hilfe eines solchen Gasrührwerkes wird zum einen das Rühren bewerkstelligt. Zum anderen kann hiermit Gas eingespeist werden, welches beispielsweise aus einem herkömmlichen Gasspeicher oder aus einem Gassack, wie oben beschrieben, stammt. Vorzugsweise weist das über das Gasrührwerk eingespeiste Gas einen hohen Anteil an Kohlendioxid auf, beispielsweise bis zu 70 % Kohlendioxid. Mit Hilfe des kohlendioxidhaltigen Gases, welches beispielsweise aus dem unteren Bereich eines Gassackes stammen kann, wird eine Pufferwirkung im Reaktor erzielt. Als Gasrührwerke eignen sich herkömmliche Gasrührwerke, die eine Revolvertrommel umfassen können, welche einzelne Schläuche, die im Kreis angeordnet sind, getrennt ansteuern. Hierdurch wird eine Kreisbewegung mit Hilfe des austretenden Gases erzielt, welches beispielsweise mit einem Druck von 3 bis 4 bar durch die Revolvertrommel und die Schläuche geleitet wird. In der Regel ist für ein solches Rührwerk eine Gasverdichtung vorteilhaft, wie sie beispielsweise mit Hilfe eines Seitenradverdichters vorgenommen werden kann.

Bevorzugterweise kann dem Reaktor I mindestens ein Endlager für Substrat, insbesondere für vergorenes Substrat, nachgeschaltet sein. Auch in diesem Endlager können ein oder mehrere Rührwerke vorgesehen sein, um eine Durchmischung zu gewährleisten. Weiterhin ist auch in einem solchen Endlager die Erzeugung von Scherkräften vorteilhaft, um Mikroorganismen abzutöten und das produzierte Gas freizusetzen. Das im Endlager vorhandene Gas kann zu einem Gasspeicher oder beispielsweise einem Motor abgeführt werden. Das Endlager kann ebenfalls mit Vorteil mit einem Gasrührwerk gerührt werden.

Bei der unvollständigen Verbrennung bzw. Verschwelung im Pyrolysator entsteht in der Regel Schwefelsäure, welche für das verbaute Material der Biogasanlage und insbesondere für einen Motor schädlich sein kann. Daher ist eine Entschwefelung des entstehenden Gases vorteilhaft. In besonders bevorzugter Weise erfolgt eine solche Entschwefelung im Endlager durch Lufteinblasung, wodurch der Schwefel reduziert wird. Zusätzlich oder alternativ dazu kann der Schwefel oder die Schwefelverbindungen durch Aktivkohlefilter und/oder Aktivkohle in Gasleitungen gebunden werden. Ein Endlager kann daher neben der eigentlichen Lagerung bzw. Zwischenlagerung von vergorenem Substrat auch zur Gasreinigung des erfindungsgemäß produzierten Biogases genutzt werden.

Ein weiterer Vorteil der erfindungsgemäßen Biogasanlage liegt darin, daß giftige Gase, die möglicherweise bei der Pyrolyse von Substraten, insbesondere bei der Holzvergasung, entstehen können, durch die Mikroorganismen im nachgeschalteten Reaktor, in welchen das entstehende Gas eingespeist wird, abgebaut werden können. Auch dies trägt zur Reinigung der Gase bei.

Es wird weiterhin ein Pyrolysator zur Bereitstellung von kohlenmonoxid- und wasserstoffhaltigem Gas beschrieben, welcher mindestens eine Zufuhreinrichtung für sauerstoffhaltiges Gas, insbesondere für im Sauerstoffgehalt angereicherte Luft, und mindestens eine weitere Gaszuleitung, insbesondere eine Biogaszuleitung, aufweist. Weiterhin wird ein Gasspeicherungs- und/oder Entmischungsbehältnis, insbesondere ein Gassack, welcher mindestens eine Zuleitung, insbesondere mit einer in etwa mittigen Endung, mindestens eine Ableitung zu einem Gasspeicher und/oder einem Gasverbraucher, insbesondere im oberen Bereich, und/oder eine Ableitung zu mindestens einem Reaktor, insbesondere im unteren Bereich, aufweist, beschrieben. Darüber hinaus wird ein Siphon zur Absperrung von Gasströmen beschrieben, welcher vorzugsweise mit Wasser flutbar ist, sowie eine Biogasanlage zur Bereitstellung von methanhaltigen Gasen aus organischen Substraten mit mindestens einem Reaktor, welche mindestens einen heizbaren und/ oder kühlbaren Substratvorratsbehälter aufweist. Bezüglich weiterer Merkmale des Pyrolysators, des Gasspeicherungs- und/oder Entmischungsbehältnisses, des Siphons bzw. dieser Biogasanlage wird auf die obige Beschreibung verwiesen.

Schließlich umfaßt die Erfindung ein Verfahren zur Herstellung von methanhaltigen Gasen aus organischen Substraten. Dieses Verfahren umfaßt die Bereitstellung von kohlenmonoxid- und wasserstoffhaltigem Gas, die Einspeisung dieses Gases in einen Reaktor, insbesondere in einen Reaktor I, und den mikrobiellen Abbau von nicht-ligninhaltigen organischen Substraten in dem Reaktor unter Bildung des methanhaltigen Gases, wobei hierbei auch die eingespeisten Gase verstoffwechselt werden. Vorzugsweise handelt es sich bei dem kohlenmonoxid- und wasserstoffhaltigem Gas um ein Pyrolysegas, insbesondere um Holzgas. In einer besonders bevorzugten Ausführungsform des Verfahrens ist dieses Gas durch einen geringen Stickstoffanteil gekennzeichnet. Vorteilhafterweise entsteht dieses Gas durch Pyrolyse von Substraten, insbesondere von organischen Substraten, wobei in besonderer Weise ligninhaltige Substrate, wie beispielsweise Holz oder Borke, bevorzugt sind. Es können jedoch auch andere Substrate, auch anorganische Substrate, eingesetzt werden, z. B. Stroh, Knochen, Kunststoffe, z. B. PVC, Autoreifen, Plastikbecher oder Ähnliches.

In einer weiteren bevorzugten Ausführungsform wird für die beschriebene Pyrolyse sauerstoffhaltiges Gas zugeführt, wobei es sich vorzugsweise um im Sauerstoffgehalt angereicherte Luft handelt. Weiterhin wird mindestens ein Biogas zugeführt, wodurch zum einen die Zusammensetzung des bei der Pyrolyse eingespeisten Gases verändert wird und zum anderen ein Kühleffekt erzielt wird. In einer besonders bevorzugten Ausführungsform des erfindungsgemäßen Verfahrens wird dieses Biogas durch mikrobiellen Abbau von nicht-ligninhaltigen Substraten in einem weiteren Reaktor, insbesondere in einem Reaktor II, bereitgestellt, welcher dem Pyrolysator vorgeschaltet ist. Bezüglich weiterer Merkmale und der konkreteren Ausgestaltung dieses erfindungsgemäßen Verfahrens wird auf die obige Beschreibung verwiesen.

Weitere Merkmale und Ausgestaltungen der Erfindung ergeben sich aus der nachfolgenden Beschreibung der Funktionsweise in Verbindung mit den Figuren und den Unteransprüchen. Hierbei können die verschiedenen Merkmale jeweils für sich oder in Kombination miteinander verwirklicht sein.

In den Figuren zeigen:
- Fig. 1: schematisch dargestellter Aufbau einer bevorzugten Ausführungsform der erfindungsgemäßen Biogasanlage,
- Fig. 2: schematisch dargestellter Aufbau einer bevorzugten Ausführungsform des Holzvergasers,
- Fig. 3: schematische Darstellung einer bevorzugten Ausführungsform eines Gassackes.
- Fig.4: schematische Darstellung einer bevorzugten Ausführungsform eines Siphons.

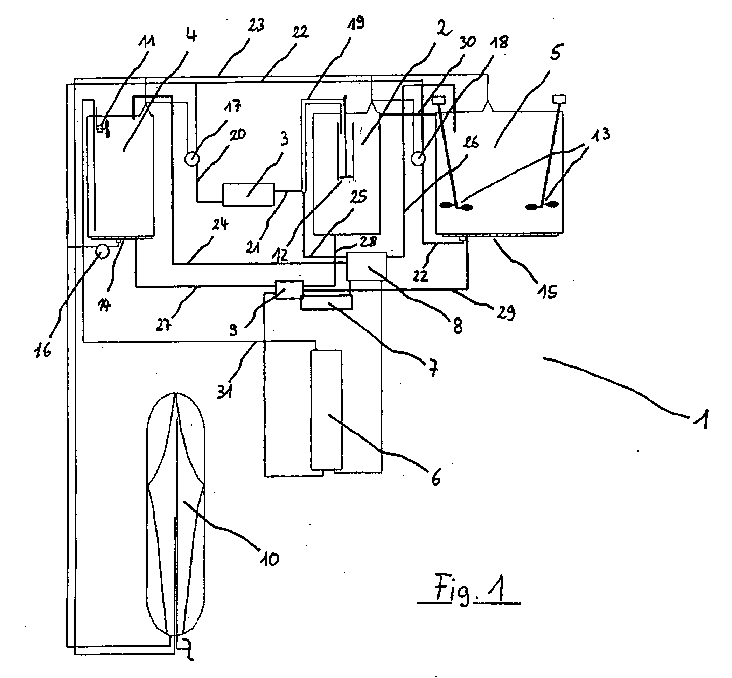

Fig. 1 zeigt in schematischer Weise den Aufbau einer bevorzugten Ausführungsform der erfindungsgemäßen Biogasanlage 1 mit einem Reaktor 2 (Reaktor I), einem Holzvergaser 3, einem Reaktor 4 (Reaktor II) und einem Endlager 5. Der Substratvorratsbehälter 6 steht über eine Pumpe 7 mit den Gülleverteiler 8 und dem Sammelschacht 9 in Verbindung. Der Biogasanlage 1 ist ein Gassack 10 zugeordnet. Im oberen Bereich des Reaktors 4 befindet sich ein mechanisches Rührwerk 11, im mittleren Bereich des Reaktors 2 befindet sich ein mechanisches Rührwerk 12. Im Endlager 5 befinden sich ebenfalls mechanische Rührwerke 13. Der Reaktor 4 und das Endlager 5 sind jeweils mit einem Gasrührwerk 14 und 15 versehen. Den Gasrührwerken 14 und 15 sowie dem Holzvergaser 3 ist jeweils ein Gasverdichter 16, 18 bzw. 17 vorgeschaltet. Der Reaktor 2 ist mit einer Substratdruckleitung 19 versehen. Zum Holzvergaser 3 läuft eine Biogasleitung 20, und zwischen dem Holzvergaser 3 und dem Reaktor 2 verläuft eine Holzgasleitung 21. Der Gassack 10 ist über Gasleitungen 22 und 23 mit den Reaktoren 2 und 4 sowie dem Endlager 5 verbunden. Vom Gülleverteiler 8 verläufen Substratzuleitungen 24, 25 und 26 zu den Reaktoren 4 und 2 sowie dem Endlager 5. Die Reaktoren 4 und 2 sowie das Endlager 5 stehen über Substratableitungen 27, 28 und 29 mit dem Sammelschacht 9 in Verbindung. Zwischen Reaktor 2 und dem Endlager 5 ist im oberen Bereich ein Überlauf 30 für Substrat, insbesondere für Gülle, angeordnet. Zwischen Substratvorratsbehälter 6 und Reaktor 4 verläuft eine Gasausgleichsleitung 31.

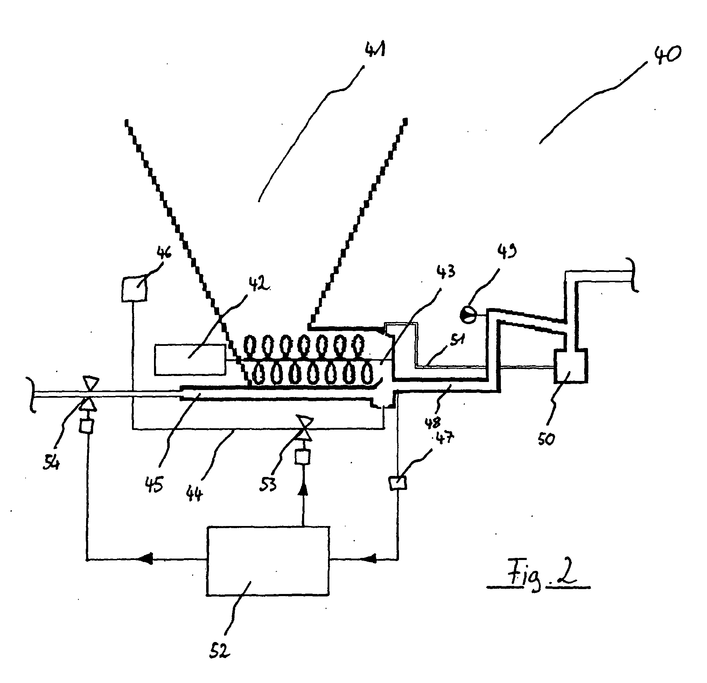

Fig. 2 zeigt in beispielhafter Ausführungsform einen Holzvergaser 40 mit einer Substrateinfülleinheit 41, einem Schneckenmotor 42 und einer Brennkammer 43. Zur Brennkammer 43 führt eine Gasleitung 44 für sauerstoffhaltiges Gas sowie eine Biogasleitung 45. Die Gasleitung 44 wird von einem Sauerstoffkonzentrator 46 gespeist. Im Bereich der Brennkammer 43 ist ein Temperaturfühler 47 angeordnet. Eine Holzgasleitung 48 ist mit einer Wassereinspritzung 49 sowie einem Kondensatfänger 50 versehen. Zwischen Kondensatfänger 50 und der Brennkammer 43 ist eine Rückflußleitung 51 angeordnet. Der Temperaturfühler 47 steht mit einer Steuereinrichtung 52 in Verbindung, die über Steuerelemente bzw. Ventile 53 und 54 mit den Gasleitungen 44 und 45 verschaltet ist.

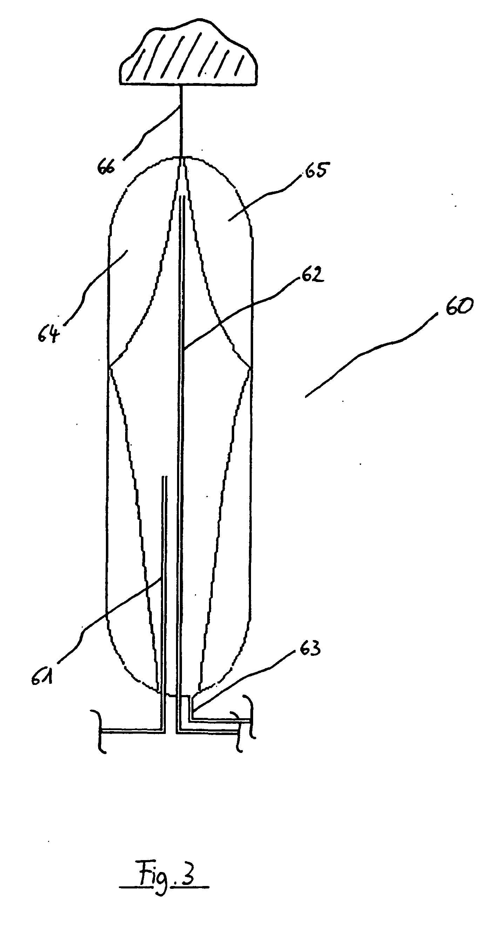

Fig. 3 zeigt in bevorzugter Ausführungsform einen Gassack 60 mit einer Gaszuleitung 61 mit in etwa mittiger Endung, einer Ableitung 62 im oberen Bereich des Gassackes 60 sowie einer weiteren Ableitung 63 im unteren Bereich des Gassackes 60. Innerhalb des Gassackes 60 sind feuchte Tücher 64 und 65 lose aufgehängt. Der Gassack 60 ist über eine Aufhängung 66 an einem stationären Teil aufgehängt.

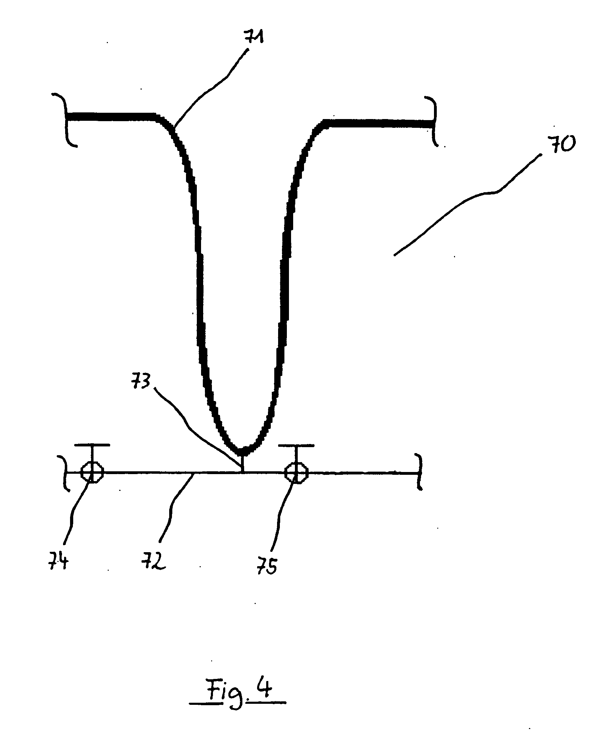

Fig. 4 zeigt eine bevorzugte Ausgestaltung eines Siphons 70 mit einer Gasleitung 71 und einer Wasserleitung 72. Die Wasserleitung 72 steht über eine Wassersäule 73 mit der Gasleitung 71 an deren tiefsten Punkt in Verbindung. Die Wasserleitung 72 ist mit Ventilen 74 und 75 versehen.

Im folgenden ist die Funktionsweise der erfindungsgemäßen Biogasanlage anhand einer beispielhaften Ausführungsform der erfindungsgemä-βen Biogasanlage (Fig. 1) dargestellt. Im Reaktor 2 befindet sich organisches Substrat, vorzugsweise Gülle, welche von anaeroben Bakterien verstoffwechselt wird. In diesen Reaktor 2 wird mittels einer Gülledruckleitung 19 Substrat und ein Gemisch aus Holzgas und Methan eingespeist, welches durch einen Holzvergaser 3 bereitgestellt wird. Dieses eingespeiste Gas setzt sich im wesentlichen aus Kohlenmonoxid (etwa 60 %), Methan (etwa 30 %) und Wasserstoffionen (etwa 10 %) zusammen. Dieses Gasgemisch wird über die Holzgasleitung 21 in die Gülledruckleitung 19 eingespeist und mit Hilfe von Unterdruck in den Reaktor 2 eingebracht. Das Kohlenmonoxid löst sich insbesondere mit Hilfe des Unterdrucks in der Gülle des Reaktors 2, was weiterhin durch ein kontinuierlich laufendes Rührwerk 12 in der Mitte des Reaktors 2 unterstützt wird. Die Temperatur im Reaktor 2 stellt sich insbesondere durch die Zuleitung des heißen Holzgasgemisches auf etwa 55 °C ein. Durch die Verstoffwechselung der Gülle und des eingeführten Gasgemisches entsteht ein sehr wertvolles Biogas, welches zu etwa 69 % aus Methan, zu etwa 30 % aus Kohlendioxid und zu etwa 1 % aus Schwefelwasserstoff besteht.

Der Holzvergaser 3 ist vorzugsweise über eine Biogasleitung 20 mit dem Reaktor 4 verbunden. In diesem Reaktor 4 findet ebenfalls eine Verstoffwechselung von organischen Substraten, insbesondere von Gülle, durch anaerobe Bakterien statt. In diesem Reaktor 4 entsteht ein Biogas, welches sich neben weiteren Bestandteilen aus etwa 45 % Methan und etwa 54 % Kohlendioxid zusammensetzt. Der Reaktor 4 ist im oberen Bereich mit einem Rührwerk 11, insbesondere mit einem Schnelläufer-Tauchmotor-Rührwerk, ausgestattet. Der untere Bereich des Reaktors 4 ist mit einem Gasrührwerk 14 mit einem davorgeschalteten Gasverdichter 16, beispielsweise einem Seitenradverdichter, versehen. Dieses Gasrührwerk 14 wird von einem Gassack 10 gespeist. Mit Hilfe dieses Gasrührwerks wird ein Gas in den Reaktor 4 eingespeist, welches einen hohen Gehalt an Kohlendioxid aufweist (etwa 70 %), wobei dieser hohe Gehalt an Kohlendioxid eine Pufferwirkung im Reaktor 4 entfaltet, so daß der pH-Wert im Reaktor gute Bedingungen für die darin enthaltenden Mikroorganismen liefert. Eine Gaseinspeisung aus dem Gassack 10 ist ebenfalls in den Holzvergaser 3 über einen Gasverdichter 17, z. B. einem Seitenradverdichter, mit Mischventil möglich.

Das vergorene Substrat aus dem Reaktor 2 kann über einen Überlauf 30 in das Endlager 5 gelangen, aus welchem es gegebenenfalls mit Hilfe einer zentralen Pumpe 7 abgezogen werden kann. Mit Hilfe der Rührwerke 13 werden in diesem Endlager Scherkräfte ausgeübt, die Bakterien zerstören und das darin produzierte und gespeicherte Biogas freisetzen. Die im Endlager 5 sowie in den Reaktoren 2 und 4 gebildeten und/ oder gelagerten Gase werden über eine Gasleitung 23 dem Gassack 10 zugeführt. Eine andere Möglichkeit ist die Ableitung der Gase zu einem weiteren oder alternativen Gasspeicher bzw. zu einem Endverbraucher wie beispielsweise einem Motor, zur letztendlichen Energiegewinnung aus dem entstandenen methanhaltigen Gas. Eine Entschwefelung der entstandenen Gase erfolgt vorzugsweise im Endlager 5 durch Lufteinblasung, was hier nicht dargestellt ist. Das Endlager 5 wird vorzugsweise über ein Gasrührwerk 15 gerührt, welches über einen Gasverdichter 18 und eine Gasleitung 22 aus dem Gassack 10 gespeist wird. Über dieses Gasrührwerk 15 wird zum einen das Material im Endlager 5 in Bewegung gehalten. Zum anderen wird hierdurch bereits gebildetes Biogas in das Endlager 5 zur Gasreinigung, insbesondere zur Entschwefelung, eingebracht.

Die Befütterung der Reaktoren 2 und 4 erfolgt über einen zentralen Substratvorratsbehälter 6, der mit entsprechenden Substratzuleitungen 24 und 25 mit den Reaktoren 2 und 4 sowie auch mit dem Endlager 5 über die Substratzuleitung 26 in Verbindung steht. Der Substratzulauf erfolgt mit Hilfe einer zentralen Pumpe 7 und dem Gülleverteiler 8. Über den Sammelschacht 9 wird überschüssiges Substrat aus dem Reaktoren 2 und 4 sowie dem Endlager 5 mit Hilfe der Pumpe 7 abgezogen. Der zentrale Substratvorratsbehälter 6 wird beispielsweise über einen Schneckenmotor oder ein Güllefaß (nicht dargestellt) extern befüllt. Das Frischsubstrat im Substratvorratsbehälter 6 wird vor der Beschickung der Reaktoren 2 und 4 erhitzt. Vorzugsweise erfolgt eine Erhitzung auf etwa 130 °C. Diese Temperatur wird für einige Minuten, beispielsweise für etwa 7 Minuten oder mehr, gehalten, wodurch eine Hygienisierung, d. h. eine Abtötung von verschiedenen Keimen, in dem Substrat erfolgt. Vor dem Beschicken der Reaktoren 2 und 4 mit dem hygienisierten Substrat ist es vorteilhaft, wenn das Substrat vorher wieder auf niedrigere Temperaturen abgekühlt wird, um eine Schädigung der Anlage, insbesondere der Pumpe 7, zu vermeiden. Die Abkühlung des Substrates kann auf etwa 70 bis 80 °C erfolgen, wobei dies durch eine Rückführung der Substrate aus den Reaktoren 2 und 4 sowie möglicherweise auch aus dem Endlager 5 erfolgt. Zwischen dem Substratvorratsbehälter 6 und dem Reaktor 4 befindet sich eine Gasausgleichsleitung 31. Hierbei handelt es sich vorzugsweise um eine Gasausgleichsdruckleitung, die durch den Druck und die Temperatur in dem zentralen Substratvorratsbehälter 6 den Flüssigkeitsstand des Reaktors 4 reguliert.

Die Ableitung des gebildeten methanhaltigen Gases zu einem Endverbraucher, beispielsweise einem Motor, zur letztendlichen Umsetzung des entstandenen Gases in elektrische Energie, erfolgt entweder direkt aus den Reaktoren 2, 4 und/oder vorzugsweise aus dem Endlager 5. Besonders vorteilhaft ist es, diese Gase zunächst beispielsweise in einem Gassack 10 zwischenzuspeichern, um sie dann mit einer entsprechenden Ableitung aus dem Gassack 10 dem Endverbraucher zuzuführen, wie weiter unten näher erläutert werden wird.

Fig. 2 stellt eine bevorzugte Ausführungsform eines Holzvergasers 40 dar, wie er mit Vorteil in einer erfindungsgemäßen Biogasanlage eingesetzt werden kann. Durch eine Pyrolyse der hierin unvollständig verbrannten ligninhaltigen Substrate, also insbesondere Holz, Borke oder Vergleichbares, entsteht das sogenannte Holzgas, welches in einen nachfolgenden Reaktor eingespeist werden kann. Es können auch andere Substrate entsprechend verbrannt werden, wobei hierfür vergleichbare Pyrolyseeinheiten zum Einsatz kommen können. Der hier dargestellte Holzvergaser 40 unterscheidet sich von herkömmlichen Holzvergasern im wesentlichen dadurch, daß nicht Luft, sondern aufbereitete Luft (aL) mit einem erhöhten Anteil von Sauerstoff zur Pyrolyse dient. Diese Pyrolyse, d. h. also eine Verschwelung von Substraten, erfolgt in der Brennkammer 43, die über einen Schneckenmotor 42 mit den Substraten, beispielsweise Holzhackschnitzeln, durch die Substrateinfülleinheit 41 mit Biomasse versehen wird. Nach einer einmaligen Zündung in der Brennkammer 43 schwelt das Substrat weiter, wobei das Holzgas gebildet wird. Für diesen Vorgang wird sauerstoffhaltiges Gas, also insbesondere die im Sauerstoffgehalt angereicherte Luft, über eine Gasleitung 44 von einem Sauerstoffkonzentrator 46 der Brennkammer 43 zugeleitet. Der Sauerstoffgehalt dieses Gases kann vorzugsweise etwa 70 % oder mehr betragen. Der Brennkammer 43 wird vorzugsweise ein weiteres Gas über die Gasleitung 45 zugeführt, wobei es sich hierbei insbesondere um Biogas aus einem Reaktor oder einem Gasspeicherungsbehältnis, beispielsweise einem Gassack 10, handelt. Mit diesem zusätzlichen Gas wird zum einen eine Kühlwirkung erzielt. Zum anderen wird hierdurch die Gaszusammensetzung in der Brennkammer 43 bestimmt. Das resultierende Gasgemisch mit einem hohen Anteil von Kohlendioxid wird über die nun glühenden Holzanteile geblasen. Hierbei reagiert das Kohlendioxid mit dem Kohlenstoff im Substrat, also insbesondere im Holz, zu Kohlenmonoxid. Im selben Verhältnis wird Wasserstoff frei. Bei diesem unvollständigen Verbrennungsvorgang ist die Temperatur ein wichtiger Faktor. Diese Temperatur darf nicht zu hoch liegen, da es sonst zu einer vollständigen Verbrennung kommt. Vorzugsweise wird die Temperatur unterhalb von 1.000 °C gehalten, insbesondere unterhalb von 800 °C. Dies kann mit Hilfe eines Sensors, insbesondere eines Temperaturfühlers 47, reguliert bzw. gesteuert werden, welcher die Zufuhr des sauerstoffhaltigen Gases und/oder die Zufuhr des Biogases bzw. des weiteren Gases über eine Steuereinrichtung 52 und Steuerelemente 53 und 54 reguliert und/oder steuert. Bei den Steuerelementen 53 und 54 handelt es sich vorzugsweise jeweils um Ventile mit einem entsprechenden Antrieb. Methan, welches über das Biogas bzw. über das weitere Gas dem Holzvergaser zugeführt wird, wird bei dem Verbrennungsvorgang nicht verbraucht und wird zusammen mit dem entstehenden Holzgas bzw. dem Pyrolysegas über die Holzgasleitung 48 abgeleitet. Die Holzgasleitung 48 ist mit einer Wassereinspritzung 49 versehen, welche zum Abfangen nicht ausgekokster Holzschwebstoffe dient, die im Gasstrom mitgerissen werden. Die Wassereinspritzung erfolgt vorzugsweise mit Hochdruck, so daß die Holzschwebstoffe im Wassernebel platzen oder sich so mit Wasser vollsaugen, daß diese in den Kondensatfänger 50 fallen. Die im Kondensatfänger 50 befindlichen Partikel bzw. das dort befindliche Wasser kann über eine Rückflußleitung 51 zurück zur Brennkammer 43 gepumpt werden. Ein solcher beispielhafter Holzvergaser stellt auf diese Weise energiereiches Holzgas mit vermindertem Stickstoff- und Stickstoffoxidgehalt bereit, welches mit großem Vorteil in einem Reaktor von anaeroben Mikroorganismen verstoffwechselt werden kann, so daß ein sehr vorteilhaftes Biogas mit hohem Methangehalt gebildet werden kann.

Fig. 3 zeigt in schematischer Weise einen beispielhaften Gassack 60, welcher als Gasspeicherungs- und Gasentmischungsbehältnis dient. Der Gassack 60 besteht im wesentlichen aus einem flexiblen Schlauch, welcher mit feuchten Tüchern 64 und 65 versehen ist. Ein solcher Gassack kann beispielsweise lose in einem Getreidesilo über die Aufhängung 66 aufgehängt sein. Die Beschickung mit Gas erfolgt über die Zuleitung 61, die etwa in der Mitte des Gassackes 60 endet. Der Sack bezweckt auf der einen Seite eine Gasspeicherung sowie auf der anderen Seite die Möglichkeit zur Gasberuhigung bzw. zur Gasentmischung. So ist der Methangehalt im oberen Teil des Sackes deutlich höher als im unteren Teil. Das Gas mit hohem Methangehalt wird über die Ableitung 62 aus dem oberen Bereich des Gassackes 60 abgeführt, so daß dieses sehr energiereiche Gas beispielsweise für einen Motor oder einen anderen Endverbraucher genutzt werden kann. Im unteren Bereich des Gassackes sammelt sich vermehrt Kohlendioxid an. Das entsprechende Gasgemisch hat eine gewisse Pufferwirkung und kann beispielsweise über Gasrührwerke in entsprechende Reaktoren eingeleitet werden. Zur Ableitung dieses Gases mit hohem Kohlendioxidgehalt ist die Ableitung 63 vorgesehen. Diese Ableitung führt beispielsweise zum Gasrührwerk 16 des Reaktors II 4. Die Entmischung des Gases wird durch lose aufgehängte befeuchtete Tücher 64 und 65 verstärkt. Vorzugsweise werden diese Tücher mit Wasser besprüht, beispielsweise mit einem handelsüblichen Hochdruckreiniger. Dieses Besprühen kann z. B. von oben erfolgen.

Fig. 4 stellt in schematischer Weise einen beispielhaften Siphon 70 dar, wie er in die Gasstrecken der erfindungsgemäßen Biogasanlage integriert sein kann. Derartige Siphons 70 werden vor allem im niedrigen Gasdruckbereich eingesetzt und dienen zum einen der Möglichkeit der Absperrung der Gasleitung. Zum anderen fördern sie eine Gastrocknung. Unterhalb der im Siphon geführten Gasleitung 71 befindet sich eine Wasserleitung 72, deren Wasserfluß über eine Pumpe gesteuert sein kann. Am tiefsten Punkt der siphonförmigen Gasleitung 71 steht das Gas mit einer Wassersäule 73 mehr oder weniger in Verbindung. Zum Absperren der Gasleitung, beispielsweise aus Sicherheitsgründen, wird die Wasserleitung stromaufwärts der Wassersäule 73 durch das Ventil 75 abgesperrt. Dies kann auch mit Hilfe einer Pumpe erfolgen, die gegebenenfalls ein Zurückpumpen des Wassers bewerkstelligt. Hierdurch staut sich das Wasser in der siphonförmigen Gasleitung zurück, eventuell unterstützt durch das Ventil 74, wodurch der Gasfluß gestoppt wird. Hierfür reicht bereits eine Wassersäule von 3,5 cm in der siphonförmigen Gasleitung 71 bei entsprechend niedrigem Gasdruck aus. Derartige Siphons 70 können an verschiedene Stellen in den Gasstrecken der erfindungsgemäßen Biogasanlage integriert sein.

## Patentansprüche

1. Biogasanlage (1) zur Bereitstellung von methanhaltigen Gasen aus organischen Substraten mit mindestens einem Reaktor I (2), **dadurch gekennzeichnet, dass** der Reaktor I (2) eine Gaszufuhreinheit für die Zufuhr von kohlenmonoxid- und wasserstoffhaltigem Gas aufweist, und die Gaszufuhreinheit ein Pyrolysator (3) mit mindestens einer Pyrotysegasteitung (21) zum Reaktor I (2) ist und dem Pyrolysator (3) ein weiterer Reaktor II (4) mit mindestens einer Biogasleitung vorgeschaltet ist.

2. Biogasanlage (1) nach Anspruch 1, **dadurch gekennzeichnet, dass** die Gaszufuhreinheit eine Pyrolysegaszufuhreinheit, insbesondere eine Holzgaszufuhreinheit, ist.

3. Biogasanlage (1) nach Anspruch 1 oder Anspruch 2, **dadurch gekennzeichnet, dass** der Pyrolysator (3) ein Holzvergaser (40) mit mindestens einer Holzgasleitung (48) zum Reaktor I (2) ist.

4. Biogasanlage (1) nach Anspruch 3, **dadurch gekennzeichnet, dass** dem Pyrolysator (3, 40) mindestens eine Zufuhreinrichtung (44) für sauerstoffhaltiges Gas, insbesondere für im Sauerstoffgehalt angereicherte Luft, zugeordnet ist.

5. Biogasanlage (1) nach Anspruch 3 oder Anspruch 4, **dadurch gekennzeichnet, dass** der Pyrolysator (3, 40) mindestens eine weitere Gaszuleitung (20, 45), insbesondere eine Biogaszuleitung, aufweist.

6. Biogasanlage (1) nach einem der Ansprüche 3 bis 5, **dadurch gekennzeichnet, dass** dem Pyrolysator (40) Steuerungs- und/oder Regelungsmittel zur Steuerung und/oder Regelung der Sauerstoffkonzentration und/oder der Temperatur im Pyrolysator zugeordnet sind.

7. Biogasanlage (1) nach Anspruch 5 oder Anspruch 6, **dadurch gekennzeichnet, dass** an der Gaszuleitung (20) des Pyrolysators (3), insbesondere der Biogaszuleitung, mindestens ein Verdichter (17) angeordnet ist.

8. Biogasanlage (1) nach einem der Ansprüche 3 bis 7, **dadurch gekennzeichnet, dass** im Bereich der Pyrolysegasleitung (48) des Pyrolysators (40) eine Wassereinspritzungseinrichtung (49) und/oder ein Kondensatfänger (50) angeordnet ist.

9. Biogasanlage (1) nach einem der Ansprüche 3 bis 8, **dadurch gekennzeichnet, dass** im Pyrolysator (3) mindestens eine Kühleinrichtung, insbesondere eine Flüssigkeitskühleinrichtung, angeordnet ist.

10. Biogasanlage (1) nach einem der vorhergehenden Ansprüche, **dadurch gekennzeichnet, dass** dem Reaktor I (2) mindestens eine Druckleitung (19) für eine Substratzufuhr zugeordnet ist, wobei vorzugsweise die Druckleitung mindestens eine Gaseinspeisung für kohlenmonoxid- und wasserstoffhaltiges Gas aufweist.

11. Biogasanlage (1) nach einem der vorhergehenden Ansprüche, **dadurch gekennzeichnet, dass** der Biogasanlage mindestens ein Gasspeicherungs- und/oder -entmischungsbehältnis, insbesondere ein Gassack (10, 60), zugeordnet ist.

12. Biogasanlage (1) nach Anspruch 11, **dadurch gekennzeichnet, dass** das Gasspeicherungs- und/oder -entmischungsbehältnis (60) mindestens eine Zuleitung (61), insbesondere mit einer in etwa mittigen Endung, mindestens eine Ableitung (62) zu einem Gasspeicher und/oder einem Gasverbraucher, insbesondere im oberen Bereich, und/oder mindestens eine Ableitung (63) zu mindestens einem Reaktor, insbesondere im unteren Bereich, aufweist.

13. Biogasanlage (1) nach Anspruch 11 oder Anspruch 12, **dadurch gekennzeichnet, dass** innerhalb des Gasspeicherungs- und/oder -entmischungsbehältnisses (60) mindestens ein anfeuchtbares flexibles Material, insbesondere ein Tuch (64, 65) angeordnet ist.

14. Biogasanlage (1) nach einem der vorhergehenden Ansprüche, **dadurch gekennzeichnet, dass** zur Absperrung von Gasströmen mindestens ein Siphon (70) vorgesehen ist, der vorzugsweise mit Wasser flutbar ist.

15. Biogasanlage (1) nach einem der vorhergehenden Ansprüche, **dadurch gekennzeichnet, dass** die Biogasanlage mindestens einen heizbaren und/oder kühlbaren Substratvorratsbehälter (6) aufweist.

16. Biogasanlage (1) nach Anspruch 15, **dadurch gekennzeichnet, dass** zwischen Substratvorratsbehälter. (6) und einem Reaktor, insbesondere Reaktor II (4), eine Gasausgleichsleitung (31) angeordnet ist.

17. Verfahren zur Herstellung von methanhaltigen Gasen aus organischen Substraten, umfassend die folgenden Verfahrensschritte:
- Bereitstellung von kohlenmonoxid- und wasserstoffhaltigem Gas, wobei das kohlenmonoxid- und wasserstoffhaltige Gas durch Pyrolyse von Substraten bereitgestellt wird,
- Einspeisung des kohlenmonoxid- und wasserstoffhaltigen Gases in einen Reaktor I (2) und
- mikrobieller Abbau von nicht-ligninhaltigen organischen Substraten im Reaktor I (2) unter Bildung des methanhaltigen Gases, **dadurch gekennzeichnet, dass** bei der Pyrolyse ein Biogas zugeführt wird, das in einem weiteren Reaktor II (4) entsteht, welcher einem Pyrolysator (3) vorgeschaltet ist.

18. Verfahren nach Anspruch 17, **dadurch gekennzeichnet, dass** das kohlenmonoxid- und wasserstoffhaltige Gas ein Pyrolysegas, insbesondere Holzgas, ist.

19. Verfahren nach Anspruch 17 oder Anspruch 18, **dadurch gekennzeichnet, dass** das kohlenmonoxid- und wasserstoffhaltige Gas einen geringen Stickstoffanteil aufweist.

20. Verfahren nach einem der Ansprüche 17 bis 19, **dadurch gekennzeichnet, dass** das kohlenmonoxid- und wasserstoffhaltige Gas durch Pyrolyse von organischen Substraten, vorzugsweise von ligninhaltigen Substraten, bereitgestellt wird.

21. Verfahren nach einem der Ansprüche 17 bis 20, **dadurch gekennzeichnet, dass** bei der Pyrolyse sauerstoffhaltiges Gas, insbesondere im Sauerstoffgehalt angereicherte Luft, zugeführt wird.

22. Verfahren nach einem der Ansprüche 17 bis 21, **dadurch gekennzeichnet, dass** das Biogas durch mikrobiellen Abbau von nicht-ligninhaltigen Substraten in dem weiteren Reaktor II (4) bereitgestellt wird.

## Claims

1. Biogas installation (1) for producing methane-containing gases from organic substrates and having at least one reactor I (2), **characterized in that** reactor I (2) has a gas supply unit for supplying gas containing carbon monoxide and hydrogen and **in that** the gas supply unit is a pyrolyser (3) with at least one pyrolysis gas line (21) to reactor I (2) and a further reactor II (4) with at least one biogas line is connected upstream of the pyrolyser (3).

2. Biogas installation (1) according to Claim 1, **characterized in that** the gas supply unit is a pyrolysis gas supply unit, in particular a wood gas supply unit.

3. Biogas installation (1) according to Claim 1 or Claim 2, **characterized in that** the pyrolyser (3) is a wood gasifier (40) with at least one wood gas line (48) to the reactor I (2).

4. Biogas installation (1) according to Claim 3, **characterized in that** at least one supply device (44) for oxygen-containing gas, in particular for air enriched in oxygen content, is assigned to the pyrolyser (3, 40).

5. Biogas installation (1) according to Claim 3 or Claim 4, **characterized in that** the pyrolyser (3, 40) has at least one further gas supply line (20, 45), in particular a biogas supply line.

6. Biogas installation (1) according to one of Claims 3 to 5, **characterized in that** control and/or regulation means are assigned to the pyrolyser (40) for control and/or regulation of the oxygen concentration and/or of the temperature in the pyrolyser.

7. Biogas installation (1) according to Claim 5 or Claim 6, **characterized in that** at least one compressor (17) is arranged on the gas supply line (20) of the pyrolyser (3), in particular on the biogas supply line.

8. Biogas installation (1) according to one of Claims 3 to 7, **characterized in that** a water injection device (49) and/or a condensate trap (50) is arranged in the area of the pyrolysis gas line (48) of the pyrolyser (40).

9. Biogas installation (1) according to one of Claims 3 to 8, **characterized in that** at least one cooling device, in particular a liquid cooling device, is arranged in the pyrolyser (3).

10. Biogas installation (1) according to one of the preceding claims, **characterized in that** at least one pressure line (19) for a substrate supply is assigned to reactor I (2), where the pressure line preferably has at least one gas feed for gas containing carbon monoxide and hydrogen.

11. Biogas installation (1) according to one of the preceding claims, **characterized in that** the biogas installation is assigned at least one gas storage and/or separation container, in particular a gas bag (10, 60).

12. Biogas installation (1) according to Claim 11, **characterized in that** the gas storage and/or separation container (60) has at least one supply line (61), in particular with an approximately central end, at least one discharge line (62) to a gas store and/or to a gas consumer, in particular in the upper area, and/or at least one discharge line (63) to at least one reactor, in particular in the lower area.

13. Biogas installation (1) according to Claim 11 or Claim 12, **characterized in that** at least one moistenable flexible material, in particular a cloth (64, 65), is arranged inside the gas storage and/or separation container (60).

14. Biogas installation (1) according to one of the preceding claims, **characterized in that** for shutting off gas flows at least one siphon (70) is provided which is preferably floodable with water.

15. Biogas installation (1) according to one of the preceding claims, **characterized in that** the biogas installation has at least one heatable and/or coolable substrate supply container (6).

16. Biogas installation (1) according to Claim 15, **characterized in that** a gas compensating line (31) is arranged between the substrate supply container (6) and a reactor, in particular reactor II (4).

17. Method for producing methane-containing gases from organic substrates, comprising the following method steps:
- producing of gas containing carbon monoxide and hydrogen, where the gas containing carbon monoxide and hydrogen is produced by pyrolysis of substrates,
- feeding of the gas containing carbon monoxide and hydrogen into a reactor I (2) and
- microbial degradation of non-lignin-containing organic substrates in reactor I (2) during formation of the methane-containing gas, **characterized in that** during pyrolysis a biogas is supplied which is generated in a further reactor II (4) connected upstream of a pyrolyser (3).

18. Method according to Claim 17, **characterized in that** the gas containing carbon monoxide and hydrogen is a pyrolysis gas, in particular wood gas.

19. Method according to Claim 17 or Claim 18, **characterized in that** the gas containing carbon monoxide and hydrogen has a low nitrogen content.

20. Method according to one of Claims 17 to 19, **characterized in that** gas containing carbon monoxide and hydrogen is produced by pyrolysis of organic substrates, preferably of lignin-containing substrates.

21. Method according to one of Claims 17 to 20, **characterized in that** during pyrolysis oxygen-containing gas, in particular air enriched in oxygen content, is supplied.

22. Method according to one of Claims 17 to 21, **characterized in that** the biogas is produced by microbial degradation of non-lignin-containing substrates in the further reactor II (4).

## Revendications

1. Installation de biogaz (1) pour préparer des gaz contenant du méthane à partir de substrats organiques avec au moins un réacteur I (2), **caractérisée en ce que** le réacteur I (2) présente une unité d'alimentation en gaz pour assurer l'alimentation en gaz contenant du monoxyde de carbone et de l'hydrogène, et l'unité d'alimentation en gaz est un pyrolyseur (3) avec au moins une conduite de gaz de pyrolyse (21) vers le réacteur I (2) et un autre réacteur II (4) avec au moins une conduite de biogaz est placé en amont du pyrolyseur (3).

2. Installation de biogaz (1) selon la revendication 1, **caractérisée en ce que** l'unité d'alimentation en gaz est une unité d'alimentation en gaz de pyrolyse, en particulier une unité d'alimentation en gaz de bois.

3. Installation de biogaz (1) selon la revendication 1 ou la revendication 2, **caractérisée en ce que** le pyrolyseur (3) est un gazéificateur de bois (40) avec au moins une conduite de gaz de bois (48) vers le réacteur I (2).

4. Installation de biogaz (1) selon la revendication 3, **caractérisée en ce qu'**au moins un dispositif d'alimentation (44) pour du gaz contenant de l'oxygène, en particulier pour de l'air enrichi en teneur en oxygène, est associé au pyrolyseur (3, 40).

5. Installation de biogaz (1) selon la revendication 3 ou la revendication 4, **caractérisée en ce que** le pyrolyseur (3, 40) présente au moins une autre conduite d'arrivée de gaz (20, 45), en particulier une conduite d'arrivée de biogaz.

6. Installation de biogaz (1) selon l'une des revendications 3 à 5, **caractérisée en ce que** des moyens de commande et/ou de régulation permettant la commande et/ou la régulation de la concentration d'oxygène et/ou de la température dans le pyrolyseur sont associés au pyrolyseur (40).

7. Installation de biogaz (1) selon la revendication 5 ou la revendication 6, **caractérisée en ce qu'**au moins un compresseur (17) est disposé sur la conduite d'arrivée de gaz (20) du pyrolyseur (3), en particulier sur la conduite d'arrivée de biogaz.

8. Installation de biogaz (1) selon l'une des revendications 3 à 7, **caractérisée en ce qu'**un dispositif d'injection d'eau (49) et/ou un capteur de condensat (50) est (sont) disposé(s) dans la zone de la conduite de gaz de pyrolyse (48) du pyrolyseur (40).

9. Installation de biogaz (1) selon l'une des revendications 3 à 8, **caractérisée en ce qu'**au moins un dispositif de refroidissement, en particulier un dispositif de refroidissement par liquide, est disposé dans le pyrolyseur (3).

10. Installation de biogaz (1) selon l'une des revendications précédentes, **caractérisée en ce qu'**au moins une conduite de pression (19) pour une alimentation en substrats est associée au réacteur I (2), sachant que la conduite de pression présente de préférence au moins une alimentation en gaz pour du gaz contenant du monoxyde de carbone et de l'hydrogène.

11. Installation de biogaz (1) selon l'une des revendications précédentes, **caractérisée en ce qu'**au moins un réservoir de stockage et/ou de ségrégation du gaz, en particulier un sac gonflable (10, 60), est associé à l'installation de biogaz.

12. Installation de biogaz (1) selon la revendication 11, **caractérisée en ce que** le réservoir de stockage et/ou de ségrégation du gaz (60) présente au moins une conduite d'alimentation (61), dont une extrémité se trouve en particulier approximativement au centre, au moins une conduite d'évacuation (62) vers un accumulateur de gaz et/ou un consommateur de gaz, en particulier dans la zone supérieure, et/ou au moins une conduite d'évacuation (63) vers au moins un réacteur, en particulier dans la zone inférieure.

13. Installation de biogaz (1) selon la revendication 11 ou la revendication 12, **caractérisée en ce qu'**au moins un matériau flexible et pouvant être humidifié, en particulier une pièce de tissu (64, 65), est disposé à l'intérieur du réservoir de stockage et/ou de ségrégation du gaz (60).

14. Installation de biogaz (1) selon l'une des revendications précédentes, **caractérisée en ce qu'**est prévu, pour couper des flux de gaz, au moins un siphon (70) pouvant de préférence être rempli d'eau.

15. Installation de biogaz (1) selon l'une des revendications précédentes, **caractérisée en ce que** l'installation de biogaz présente au moins un réservoir de stockage de substrats (6) pouvant être chauffé et/ou réfrigéré.

16. Installation de biogaz (1) selon la revendication 15, **caractérisée en ce qu'**une conduite d'équilibrage de gaz (31) est disposée entre le réservoir de stockage de substrats (6) et un réacteur, en particulier le réacteur II (4).

17. Procédé de fabrication de gaz contenant du méthane à partir de substrats organiques, comprenant les étapes suivantes :
- obtention de gaz contenant du monoxyde de carbone et de l'hydrogène, sachant que le gaz contenant du monoxyde de carbone et de l'hydrogène est produit par pyrolyse de substrats,
- injection du gaz contenant du monoxyde de carbone et de l'hydrogène dans un réacteur I (2) et
- dégradation microbienne de substrats organiques ne contenant pas de lignine dans le réacteur I (2) sous formation du gaz contenant du méthane, **caractérisé en ce que** lors de la pyrolyse est injecté un biogaz qui est produit dans un autre réacteur II (4) placé en amont d'un pyrolyseur (3).

18. Procédé selon la revendication 17, **caractérisé en ce que** le gaz contenant du monoxyde de carbone et de l'hydrogène est un gaz de pyrolyse, en particulier un gaz de bois.

19. Procédé selon la revendication 17 ou la revendication 18, **caractérisé en ce que** le gaz contenant du monoxyde de carbone et de l'hydrogène présente une faible teneur en azote.

20. Procédé selon l'une des revendications 17 à 19, **caractérisé en ce que** le gaz contenant du monoxyde de carbone et de l'hydrogène est obtenu par pyrolyse de substrats organiques, de préférence de substrats contenant de la lignine.

21. Procédé selon l'une des revendications 17 à 20, **caractérisé en ce que** lors de la pyrolyse est injecté du gaz contenant de l'oxygène, en particulier de l'air enrichi en teneur en oxygène.

22. Procédé selon l'une des revendications 17 à 21, **caractérisé en ce que** le biogaz est obtenu par dégradation microbienne de substrats ne contenant pas de lignine dans un autre réacteur II (4).
